# EUROPÄISCHE PATENTSCHRIFT

(11) **EP 1 268 456 B1**
(45) Veröffentlichungstag und Bekanntmachung des Hinweises auf die Patenterteilung: **23.02.2005**
(21) Anmeldenummer: 01938025.2
(22) Anmeldetag: 19.03.2001
(51) Int. Cl.: C07D 301/12

(54) **VERFAHREN ZUR UMSETZUNG EINER ORGANISCHEN VERBINDUNG MIT EINEM HYDROPEROXID**
METHOD FOR CONVERTING AN ORGANIC COMPOUND WITH A HYDROPEROXIDE
PROCEDE POUR FAIRE REAGIR UN COMPOSE ORGANIQUE AVEC UN HYDROPEROXYDE

(30) Priorität: 28.03.2000 DE 10015246
(43) Veröffentlichungstag der Anmeldung: 02.01.2003
(73) Patentinhaber: BASF AKTIENGESELLSCHAFT, 67056 Ludwigshafen (DE)
(72) Erfinder: MÜLLER, Ulrich, 67435 Neustadt (DE); TELES, Joaquim, Henrique, 67166 Otterstadt (DE); RIEBER, Norbert, 68259 Mannheim (DE); WALCH, Andreas, 74193 Schwaigern (DE); BASSLER, Peter, 68519 Viernheim (DE); REHFINGER, Alwin, 67112 Mutterstadt (DE); BERG, Anne, 2170 Merksem (BE); HARDER, Wolfgang, 69469 Weinheim (DE)
(74) Vertreter: Isenbruck, Günter, Dr.
(86) Internationale Anmeldenummer: PCT/EP2001/003143
(87) Internationale Veröffentlichungsnummer: WO 2001/072729

(56) Entgegenhaltungen:
- US-A- 2 338 881
- US-A- 3 329 603
- US-A- 3 346 658
- US-A- 3 591 629
- US-A- 4 973 778
- US-A- 5 059 738
- US-A- 5 349 072
- US-A- 5 489 726
- US-A- 5 849 937
- US-A- 5 912 367

## Beschreibung

Die vorliegende Erfindung betrifft ein Verfahren zur Umsetzung einer organischen Verbindung mit einem Hydroperoxid, wobei innerhalb des Verfahrens mindestens zwei parallel geschaltete Reaktoren eingesetzt werden. Weiter betrifft die Erfindung die Vorrichtung, in der das Verfahren ausgeführt wird, selbst.

Umsetzungen von organischen Verbindungen mit Hydroperoxiden, d.h. mit Verbindungen der allgemeinen Formel ROOH, werden in den gängigen Verfahren des Standes der Technik im Regelfall einstufig durchgeführt.

Die US-A-5,262,550 beschreibt beispielsweise ein Verfahren zur Epoxidierung von Alkenen, in dem in einer Stufe Alken mit Wasserstoffperoxid oder einem Wasserstoffperoxid-Precursor zum entsprechenden Alkenoxid umgesetzt wird.

Die US-A-4,883,260 offenbart ein Verfahren, in dem Alken mit Wasserstoffperoxid in einer Stufe im Stahlautoklaven bzw. im Glasautoklaven umgesetzt wird.

Die US 5,912,367 beschreibt ein Verfahren zur Herstellung von Propylenoxid umfassend (a) das Inkontaktbringen von Propen mit einer aktiven Sauerstoffspezies in flüssiger Phase, wobei ein heterogener Katalysator eingesetzt wird, und weiter umfassend (b) das gleichzeitige Erhöhen der Temperatur und des Drucks, so dass in der flüssigen Phase eine im wesentlichen konstante Propenkonzentration aufrecht erhalten bleibt, wobei in (a) und (b) eine bestimmte Minimalausbeute an Propylenoxid erreicht werden soll. Als aktive Sauerstoffspezies sind unter anderem Wasserstoffperoxid oder organische Hydroperoxide genannt, als heterogener Katalysator beispielsweise ein Titansilikalit.

In S.-H. Wang, Process Economics Program, Report 2E, S. 6-1 bis 6-27, SRI International (1994) ist beispielsweise ein Verfahren beschrieben, in dem in einer Stufe eine ca. 17 Gew.-%ige Ethylbenzolhydroperoxidlösung mit Propen an einem homogenen Mo-Katalysator umgesetzt wird.

Die gleiche Schrift offenbart auf den Seiten 6-28 bis 6-47 ein Verfahren, in dem in einer Stufe eine ca. 20 Gew.-%ige Ethylbenzolhydroperoxidlösung mit Propen an einem heterogenen Ti/SiO₂-Katalysator umgesetzt wird, wobei das Alken epoxidiert wird.

Ebenfalls in dieser Schrift wird auf den Seiten 5-1 bis 5-21 ein Verfahren offenbart, in dem in einer Stufe eine ca. 40 Gew.-%ige tert-Butylhydroperoxidlösung mit Propen an einem homogenen Mo-Katalysator umgesetzt wird, wobei das Alken epoxidiert wird.

Auch zweistufige Verfahren sind aus dem Stand der Technik bekannt.

Die US 5,349,072 beschreibt ein Verfahren zur Herstellung von Propylenoxid und Tertiärbutylalkohol, in dem Tertiärbutylhydroperoxid, gelöst in Tertiärbutylalkohol, mit Propen in Anwesenheit eines löslichen Molybdän-Katalysators umgesetzt wird. Die Reaktion wird stufenweise in einem Reaktorsystem durchgeführt, das ein erstes isothermes Segment und ein zweites adiabatisches Segment aufweist. In dem ersten isothermen Segment wird eine bestimmte Menge des Tertiärbutylhydroperoxids umgesetzt, wodurch eine erste Mischung entsteht, von der ein Recycling-Strom abgezweigt und in das isotherme Segment rückgeführt wird. Die restliche Mischung wird durch das adiabatische Segment geführt, wobei nochmals ein bestimmter Anteil des Tertiärbutylhydroperoxids umgesetzt wird.

In der oben genannten SRI-Schrift ist beispielsweise auf den Seiten 5-22 bis 5-43 ein Verfahren offenbart, in dem in zwei direkt aufeinanderfolgenden Stufen eine ca. 72 Gew.-%ige tert-Butylhydroperoxidlösung mit Propen und einem homogenen Mo-Katalysator umgesetzt wird, wobei das Alken epoxidiert wird.

Die US-A 5,849,937 beschreibt ein Verfahren zur Epoxidierung eines Olefins, wobei eine Kaskade von mindestens zwei Festbettreaktoren eingesetzt wird, wobei diese Festbettreaktoren hintereinander geschaltet, also seriell miteinander verbunden sind.

Eine Aufgabe der vorliegenden Erfindung war es, ein Verfahren bereitzustellen, das es erlaubt, einen hohen Durchsatz an Eduktströmen zu gewährleisten, wobei das Verfahren im besonderen so ausgelegt sein sollte, daß auch die Regenerierung von Katalysatoren, die bei der Umsetzung von organischer Verbindung mit Hydroperoxid verwendet werden, in einfacher Art und Weise durchführbar ist.

Demgemäß betrifft die vorliegende Erfindung ein kontinuierliches Verfahren zur Umsetzung einer organischen Verbindung mit Hydroperoxid in Gegenwart eines Katalysators, wobei das Verfahren eine Reaktionsstufe (R1) aufweist, in der die organische Verbindung in Anwesenheit eines Katalysators unter Erhalt eines Produktstroms (P1) umgesetzt wird, und in Reaktionsstufe (R1) eine Reaktoranordnung gewählt wird, die zwei oder mehr parallel geschaltete Reaktoren umfaßt. Ebenso kann (R1) eine Reaktoranordnung aufweisen, die mindestens zwei parallel geschaltete Reaktorkaskaden aufweist, wobei jede Reaktorkaskade mindestens zwei hintereinander geschaltete Reaktoren aufweist. Weiter sind auch Reaktoranordnungen denkbar, in denen mindestens eine derartige Reaktorkaskade mit mindestens einem weiteren Reaktor parallel geschaltet ist.

Neben der mindestens einen Reaktionsstufe (R1) weist das erfindungsgemäße Verfahren auch mindestens eine Zwischenbehandlung (Z1) auf, in der der mindestens eine Produktstrom (P1) in geeigneter Weise behandelt wird.

Erfindungsgemäß weist daher das Verfahren eine wie oben beschriebene Reaktionsstufe (R1) auf, in der die organische Verbindung in Anwesenheit eines Katalysators unter Erhalt eines Produktstroms (P1) umgesetzt wird und der Produktstrom einer Zwischenbehandlung (Z1) zugeführt wird. Der Produktstrom (PZ1), der aus der Zwischenbehandlung (Z1) resultiert, wird einer weiteren Reaktionsstufe (R2) zugeführt. Weiter kann der Produktstrom (PZ1) auch beispielsweise in geeigneter Weise in mindestens zwei Teilströme geteilt werden und mindestens einer der Teilströme in (R1) rückgeführt werden, um beispielsweise nicht-umgesetzte organische Verbindung oder/und nicht-umgesetztes Hydroperoxid umzusetzen, und mindestens einer der Teilströme der Reaktionsstufe (R2) zugeführt werden.

Prinzipiell kann in (R2) jede beliebige Reaktion, die unter Verwendung des Produktstroms (PZ1) oder des mindestens einen Teilstromes von (PZ1) durchführbar ist, erfolgen. Bevorzugt wird im Rahmen des erfindungsgemäßen Verfahrens der Reaktionsstufe (R2) der Produktstrom (PZ1), der Hydroperoxid umfaßt, zugeführt und in (R2) Hydroperoxid mit der organischen Verbindung in Anwesenheit eines Katalysators unter Erhalt eines Produktstromes (P2) umgesetzt.

Demgemäß betrifft die vorliegende Erfindung ein kontinuierliches Verfahren, wie oben beschrieben, zur Umsetzung einer organischen Verbindung mit Hydroperoxid in Gegenwart eines Katalysators, wobei
- die organische Verbindung in einer Reaktionsstufe (R1) mit Hydroperoxid in Anwesenheit eines Katalysators unter Erhalt mindestens eines Produktstroms (P1) umgesetzt wird,
- der mindestens eine Produktstrom (P1) einer Zwischenbehandlung (Z1) zugeführt wird, wobei aus (Z1) mindestens ein Produktstrom (PZ1), umfassend Hydroperoxid, erhalten wird, und
- der mindestens eine Produktstrom (PZ1) einer Reaktionsstufe (R2) zugeführt wird, in der Hydroperoxid mit der organischen Verbindung in Anwesenheit eines Katalysators unter Erhalt mindestens eines Produktstroms (P2) umgesetzt wird,
dadurch gekennzeichnet, daß in den Reaktionsstufen (R1) und (R2) jeweils eine Reaktoranordnung, umfassend mindestens zwei parallel geschaltete Reaktoren, eingesetzt wird.

Die vorliegende Erfindung umfaßt damit Ausführungsformen, in denen in Reaktionsstufe (R1) zwei oder mehr parallel geschaltete Reaktoren und in Stufe (R2) zwei oder mehr parallel geschaltete Reaktoren eingesetzt werden, wobei die Anzahl der in Stufe (R1) eingesetzten und parallel geschalteten Reaktoren gleich oder unterschiedlich von der Anzahl der in Stufe (R2) eingesetzten und parallel geschalteten Reaktoren sein kann.

Die Anzahl der Eduktströme, die in den mindestens einen Reaktor in (R1) eingeleitet werden, kann im wesentlichen beliebig gewählt werden. Sämtliche Edukte können beispielsweise als jeweils separater Strom in den mindestens einen Reaktor eingeleitet werden. Ebenso können die Eduktströme vor dem Einleiten in den mindestens einen Reaktor in geeigneter Weise zusammengefaßt und der resultierende, mindestens eine Strom in den mindestens einen Reaktor eingeleitet werden. Beispielsweise ist es denkbar, in einen Reaktor aus (R1) einen Eduktstrom einzuleiten, der bereits die Zusammensetzung desjenigen Gemisches aufweist, das in dem Reaktor zur Reaktion gebracht wird.

Ganz allgemein kann der mindestens eine Eduktstrom, bevor er in einen Reaktor der Stufe (R1) eingeleitet wird, vorbehandelt werden. Hinsichtlich dieser Vorbehandlung sind unter anderem sämtliche Methoden denkbar. Beispielsweise kann zu einem oder auch mehreren Edukströmen, die in (R1) eingesetzt werden, mindestens eine basische, mindestens eine neutrale, mindestens eine saure Verbindung oder ein Gemisch aus zwei oder mehr dieser Verbindungen zugegeben werden und bevorzugt eine basische Verbindung zugegeben werden, wie dies unter anderem untenstehend im Rahmen der Zwischenbehandlung (Z1) diskutiert wird.

Je nachdem, wieviele Reaktoren in Stufe (R1) eingesetzt werden, resultieren aus der Stufe (R1) unterschiedlich viele Produktströme, die der Zwischenbehandlung (Z1) zugeführt werden. Dabei ist es beispielsweise denkbar, einen Produktstrom aus (R1) in geeigneter Weise in mehrere Ströme aufzuteilen und mehreren Vorrichtungen, in denen die Zwischenbehandlung (Z1) durchgeführt wird, zuzuführen. Ebenso können mehrere Produktströme aus (R1) zu einem oder mehreren Strömen zusammengefaßt werden und einer oder mehreren Vorrichtungen zugeführt werden, in denen die Zwischenbehandlung (Z1) durchgeführt wird.

Was die Zwischenbehandlung (Z1) anbelangt, so können ganz allgemein sämtliche denkbaren Kombinationen von gleichen oder voneinander verschiedenen Vorrichtungen in serieller und/oder paralleler Anordnung verwendet werden.

Unter dem Begriff "Zwischenbehandlung" werden im Rahmen der vorliegenden Anmeldung sämtliche Behandlungen verstanden, durch die in geeigneter Weise auf die chemischen oder physikalischen Eigenschaften eines Produktstromes aus der Reaktionsstufe (R1) Einfluß genommen wird, wobei, im Gegensatz zu den Reaktionsstufen (R1) und (R2), die Umsetzung von organischer Verbindung mit Hydroperoxid zwar erfolgen kann, jedoch nur eine untergeordnete Rolle spielt. Als Zwischenbehandlung sind demgemäß beispielsweise Zugabe einer oder mehrerer Verbindungen zu einem Produktstrom, Abtrennung einer oder mehrerer Verbindungen aus einem Produktstrom oder etwa Änderung der Temperatur oder Änderung des Druckes oder Änderung des Aggregatzustandes eines Produktstromes oder Änderung des Aggregatzustandes mindestens einer in einem Produktstrom enthaltenen chemischen Verbindung denkbar. Ebenso sind beispielsweise Mischvorgänge als Zwischenbehandlung denkbar, durch die beispielsweise die Homogenität eines Produktstroms beeinflußt werden kann.

Wird die Zwischenbehandlung beispielsweise in einer eigens für die Zwischenbehandlung vorgesehen Vorrichtung, beispielsweise in einem Reaktor, einer Abtrennvorrichtung, einer Mischvorrichtung oder ähnlichem durchgeführt, so ist die Anzahl dieser Vorrichtungen von der Anzahl der in Stufe (R1) und der Anzahl der in Stufe (R2) eingesetzten Reaktoren prinzipiell unabhängig.

Werden demgemäß beispielsweise in Stufe (R1) zwei oder mehr parallel geschaltete Reaktoren eingesetzt, so können die Produktströme aus diesen Reaktoren in geeigneter Weise zu einem einzigen Strom zusammengefaßt werden und dieser eine Strom der Zwischenbehandlung in einer einzigen Vorrichtung zugeführt werden. Ebenso ist es denkbar, die zwei oder mehr Produktströme nicht zusammenzufassen oder in geeigneter Weise zu zwei oder mehr Strömen zusammenzufassen und jeden dieser Ströme der Zwischenbehandlung in einer separaten Vorrichtung zuzuführen.

Demgemäß betrifft die Erfindung auch eine Vorrichtung zur Durchführung des erfindungsgemäßen Verfahrens, umfassend mindestens zwei parallel geschaltete Reaktoren für die Reaktionsstufe (R1), mindestens eine für die Zwischenbehandlung (Z1) vorgesehene Vorrichtung ausgewählt aus der Gruppe umfassend Reaktor, Abtrennvorrichtung oder Mischvorrichtung, und mindestens zwei parallel geschaltete Reaktoren für die Reaktionsstufe (R2).

Weiter umfaßt die vorliegende Erfindung Ausführungsformen, in denen die Zwischenbehandlung (Z1) in mindestens zwei Vorrichtungen durchgeführt wird, die seriell geschaltet sind, wobei die Vorrichtungen gleich oder verschieden voneinander sein können. Ebenso sind Ausführungsformen umfaßt, in denen mindestens ein Produktstrom (P1) in (Z1) in einer oder mehreren parallel geschalteten Vorrichtungen zwischenbehandelt wird und mindestens ein weiterer Produktstrom (P1) in mindestens einer seriellen Anordnung von Vorrichtung gemäß (Z1) zwischenbehandelt wird. Weiter sind Ausführungsformen umfaßt, in denen mindestens zwei Produktströme jeweils separat in einer eigenen Vorrichtung in einem ersten Schritt der Zwischenbehandlung (Z1) zwischenbehandelt werden und die aus diesem Schritt in der ersten Vorrichtung resultierenden Ströme in geeigneter Weise in mindestens einer weiteren Vorrichtung, die gleich oder unterschiedlich der ersten Vorrichtung sein kann, zusammengeführt und einem weiteren Schritt von (Z1) zwischenbehandelt werden.

Unabhängig davon, in wievielen Vorrichtungen die Zwischenbehandlung (Z1) durchgeführt wird, kann die Reaktionsstufe (R2) in zwei oder mehr parallel geschalteten Reaktoren durchgeführt werden.

Diesbezüglich ist es denkbar, daß in jeden Reaktor der Stufe (R2) ein oder auch mehrere Eduktströme, die als Produktströme (PZ1) aus der Zwischenbehandlung (Z1) resultieren, eingeleitet werden, wobei ein Strom, der als Produktstrom aus der Zwischenbehandlung (Z1) resultiert, mit mindestens einem weiteren Produktstrom (PZ1) in geeigneter Weise zusammengefaßt oder wobei ein Strom (PZ1) in mindestens zwei Ströme aufgetrennt werden kann.

Unter anderem umfaßt die vorliegende Erfindung damit eine Ausführungsform, in der beispielsweise zu x (x > 1) Eduktströmen, umfassend mindestens Hydroperoxid, organische Verbindung und Lösungsmittel, mindestens eine Verbindung, beispielsweise eine basische Verbindung, zugegeben wird, die resultierenden Ströme in jeweils einen Reaktor der Reaktionsstufe (R1) geleitet werden, wobei (R1) damit x parallel geschaltete Reaktoren aufweist, der Produktstrom (P1) aus jedem der Reaktoren aus (R1) einer Zwischenbehandlung (Z1) unterworfen wird, wobei beispielsweise wiederum mindestens eine Verbindung, die gleich oder verschieden von der Verbindung sein kann, die den Eduktströmen zugesetzt wird, separat jedem Produktstrom aus (R1) zugesetzt wird, und jeder der resultierenden x Ströme (PZ1) jeweils in einen Reaktor der Reaktionsstufe (R2), die damit x parallel geschaltete Reaktoren aufweist, eingeleitet wird.

Die vorliegende Erfindung umfaßt beispielsweise ebenso eine Ausführungsform, in der zu x Eduktströmen, umfassend mindestens Hydroperoxid, organische Verbindung und Lösungsmittel, mindestens eine Verbindung, beispielsweise eine basische Verbindung, zugegeben wird, die resultierenden Ströme in jeweils einen Reaktor der Reaktionsstufe (R1) geleitet werden, wobei (R1) damit x parallel geschaltete Reaktoren aufweist, die Produktströme (P1) zu einem Strom zusammengefaßt werden und der resultierende Strom einer Zwischenbehandlung (Z1) zugeführt wird, der aus der Zwischenbehandlung resultierende Strom (PZ1) der Reaktionsstufe (R2), die einen Reaktor aufweist, zugeführt wird.

Zusätzlich zu dem mindestens einen Strom (PZ1) kann in den mindestens einen Reaktor der Stufe (R2) generell zusätzlich mindestens ein weiterer Eduktstrom eingeleitet werden, der beispielsweise mindestens ein Lösungsmittel oder organische Verbindung oder mindestens ein Hydroperoxid oder ein Gemisch aus zwei oder mehr davon umfaßt.

Als parallel geschaltete Reaktoren werden im Rahmen der vorliegende Erfindung auch Anordnungen verstanden, in denen mindestens einer der parallelen Zweige der Anordnung mindestens zwei seriell angeordnete Reaktoren aufweist. Weist eine Reaktionsstufe keine parallele angeordneten Reaktoren auf, so sind bezüglich dieser Reaktionsstufe Anordnungen denkbar. in denen mindestens zwei Reaktoren in Serie hintereinander geschaltet sind.

Je nach Dauer der Umsetzung von organischer Verbindung mit Hydroperoxid in Anwesenheit eines Katalysators wird die Katalysatoraktivität oder/und die Katalysatorselektivität auf Werte abfallen, die hinsichtlich einer ökonomischen Prozeßführung nicht mehr vertretbar sind. Im cinem solchen Fall wird der Katalysator, der verminderte Aktivität oder/und Selektivität aufweist, regeneriert.

Daher betrifft die vorliegende Erfindung auch ein Verfahren, wie oben beschrieben, dadurch gekennzeichnet, daß mindestens einer der in den mindestens zwei parallelen Reaktoren enthaltenen Katalysatoren während des Verfahrens regeneriert wird.

Hinsichtlich der Regenerierung sind sämtliche mögliche Verfahrensführungen denkbar. Insbesondere werden die in der WO 98/55228, EP-A 0 790 075, EP-A 0 743 094, JP-3-114536, Proc. 7^{th} Intern. Zeolite Conf., Tokyo, 1986, S. 129 ff, insbesondere S. 134 und 135, sowie WO 98/18556 beschriebenen Verfahren zur Regenerierung eingesetzt, die durch Bezugnahme in den Kontext der vorliegenden Anmeldung vollumfänglich einbezogen werden.

Im besonderen wird jedoch darauf geachtet, daß das kontinuierliche Verfahren zur Umsetzung von organischer Verbindung mit Hydroperoxid nicht unterbrochen werden muß, da sonst der Verfahrensdurchsatz zum Teil erheblich vermindert werden kann.

In einer bevorzugten Ausführungsform betrifft daher die vorliegende Erfindung auch ein Verfahren. wie oben beschrieben, dadurch gekennzeichnet, daß die Katalysator-Regenerierung derart durchgeführt wird, daß mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Reaktionsstufe immer mindestens ein Reaktor zur Umsetzung von organischer Verbindung mit Hydroperoxid zur Verfügung steht.

Bezüglich der prinzipiellen Durchführung einer derartigen Regenerierung unter Weiterführung der Umsetzung wird Bezug genommen auf die US 5,849,937.

Werden, wie oben beschrieben, in einer Reaktionsstufe beispielsweise in einem parallelen Zweig der Reaktionsstufe zwei oder mehr seriell angeordnete Reaktoren eingesetzt, so ist es denkbar, den gesamten parallelen Zweig aus der Reaktionsstufe abzukoppeln. Ebenso kann auch nur einer der seriell angeordneten Reaktoren aus dem Verfahren abgekoppelt werden, um den darin enthaltenen Katalysator zu regenerieren. Werden beispielsweise in einer speziellen Anordnung aus einer Reaktionsstufe soviele der Reaktoren zur Regenerierung der darin enthaltenen Katalysatoren abgekoppelt, daß nur einer der parallelen Zweige im kontinuierlichen Verfahren verbleibt, so verbleiben besonders bevorzugt im Falle, daß der verbliebene Zweig aus einer seriellen Anordnung von Reaktoren besteht, sämtliche der seriell angeordneten Reaktoren im Verfahren, damit das kontinuierliche Verfahren nicht unterbrochen wird.

Die Zeit, die ein Katalysator im Rahmen der vorliegenden Erfindung zur Umsetzung von organischer Verbindung mit Hydroperoxid in Betrieb bleibt, bevor der Reaktor aus der betreffenden Reaktionsstufe zur Regenerierung des Katalysators abgekoppelt wird, ist im Rahmen des erfindungsgemäßen Verfahrens beliebig wählbar. Bevorzugt beträgt diese Betriebszeit mindestens 500 Stunden, weiter bevorzugt mindestens 700 Stunden, insbesondere bevorzugt mindestens 900 Stunden und besonders bevorzugt mindestens 1100 Stunden. Unter dem Begriff "Betriebszeit" wird im Rahmen der vorliegenden Erfindung diejenige Zeitspanne verstanden, in der in dem betreffenden Reaktor organische Verbindung mit Hydroperoxid umgesetzt wird.

Die Zeit, in der der Reaktor zur Regenerierung des Katalysators, der in diesem Reaktor enthalten ist, aus der betreffenden Reaktionsstufe entfernt wird, liegt bevorzugt bei weniger als 350 Stunden, weiter bevorzugt bei weniger als 300 Stunden und besonders bevorzugt bei weniger als 250 Stunden. Diese Regenerierungszeit umfaßt die gesamte Zeitspanne zwischen dem Zeitpunkt, in dem der Reaktor aus der betreffenden Reaktionsstufe abgekoppelt und dem Zeitpunkt, in dem der Reaktor, enthaltend den regenerierten Katalysator, wieder in die betreffende Reaktionsstufe eingekoppelt wird.

In einer weiter bevorzugten Ausführungsform wird das erfindungsgemäße Verfahren so durchgerührt, daß zur Regenerierung des Katalysators immer nur ein einziger der parallel geschalteten Reaktoren einer Reaktionsstufe aus dem Verfahren abgekoppelt wird.

Demgemäß betrifft die vorliegende Erfindung auch ein Verfahren, wie oben beschrieben, dadurch gekennzeichnet, daß von x parallel geschalteten Reaktoren einer Reaktionsstufe immer x-1 Reaktoren zur Umsetzung von organischer Verbindung mit Hydroperoxid zur Verfügung stehen.

Hierbei ist x wie oben definiert, wobei x > 1.

Der Begriff "parallel geschalteter Reaktoren" bezeichnet im Rahmen der vorliegenden Erfindung auch Anordnungen, in denen mindestens einer der parallel geschalteten Reaktoren nicht nur einen, sondern zwei oder mehr miteinander verbundene Reaktoren aufweist. Insbesondere umfaßt der Begriff "parallel geschaltete Reaktoren" Anordnungen, in denen mindestens einer der parallel geschalteten Reaktoren eine serielle Anordnung von zwei oder mehr Reaktoren aufweist.

Unter anderem bevorzugt ist im Rahmen der vorliegenden Erfindung beispielsweise ein Verfahren, in dem die Reaktionsstufe (R1) x parallel geschaltete Reaktoren aufweist, jeder Produktstrom aus diesen Reaktoren separat einer Zwischenbehandlung (Z1) zugeführt wird und jeder Strom (PZ1) aus jeder Zwischenbehandlung jeweils in einen von x parallel geschalteten Reaktoren der Stufe (R2) geleitet wird.

Dieses Verfahren wird bevorzugt derart durchgeführt, daß zunächst in einem der parallelen Zweige Hydroperoxid mit organischer Verbindung umgesetzt wird, nach einem bestimmten Zeitintervall ein zweiter der parallelen Zweige in Betrieb genommen wird, nach einem bestimmten Zeitintervall der dritte, falls vorhanden, der parallelen Zweige in Betrieb genommen wird. Die Zeitintervalle bei diesem zeitversetzten Inbetriebnehmen werden ganz besonders bevorzugt so gewählt, daß jeder der parallelen Zweige dann aus dem Verfahren genommen werden kann, wenn der in den jeweiligen Reaktoren enthaltene Katalysator nicht mehr die erforderliche Aktivität oder/und Selektivität aufweist, die anderen Katalysatoren jedoch noch ausreichend aktiv oder/und selektiv sind. Durch Kenntnis der Katalysatoreigenschaften und damit der Möglichkeit, die angesprochenen Zeitintervalle genau zu bestimmen und beispielsweise Reaktionsbedingungen, Reaktorgrößen oder/und Anzahl der verwendeten parallelen Zweige mit den Regenerierungszeiten des Katalysators abzustimmen, ist es bei diesem zeitversetzten Inbetriebnehmen dem Fachmann möglich, ein besonders effizientes Verfahren zu betreiben, in dem besonders bevorzugt von x parallelen Zweigen immer x-1 Zweige effektiv und effizient zur Verfügung stehen. Selbstverständlich ist dieses zeitversetzte Arbeiten auch dann möglich, wenn nur eine der Realctionsstufen (R1) und (R2) x parallel geschaltete Reaktoren aufweist.

Selbstverständlich sind die Vorrichtung und das Verfahren, wie im Rahmen der vorliegenden Erfindung beschrieben, nicht auf eine Reaktionsstufe (R1), eine Zwischenbehandlung (Z1) und eine Reaktionsstufe (R2) beschränkt. Weitere Reaktionsstufen und weitere Zwischenbehandlungsstufen sind ebenfalls in sämtlichen Kombinationen umfaßt. Demgemäß kann sich beispielsweise direkt an Reaktionsstufe (R2) eine Reaktionsstufe (R3) und an diese wiederum eine Reaktionsstufe (R4) anschließen. Ebenso kann sich an (R2) eine Zwischenbehandlung (Z2) und an diese eine weitere Zwischenbehandlung (Z3) mit nachfolgender Reaktionsstufe (R3) oder an (Z2) direkt eine Reaktionsstufe (R3) anschließen. Die Art der Zwischenbehandlungen kann gleich der von (Z1) sein oder sich von (Z1) unterscheiden. Was die Reaktionsstufen (R3) und (R4) und so weiter anbelangt, so können in diesen Stufen ebenfalls Umsetzungen von Hydroperoxid mit organischer Verbindung stattfinden, wobei aber auch andere Umsetzungen beziehungsweise Reaktionen denkbar sind. Unter anderem kann beispielsweise in Stufe (R3) die Vernichtung von überschüssigem Hydroperoxid vorgesehen werden.

Demgemäß betrifft die vorliegende Erfindung auch ein Verfahren, wie oben beschrieben, umfassend mindestens eine weitere Zwischenbehandlung und mindestens eine weitere Reaktionsstufe.

Hinsichtlich der Reaktoranordnungen in den weiteren Reaktionsstufen oder der Anordnung der Vorrichtungen in den weiteren Zwischenbehandlungen sind sämtliche denkbaren Ausführungsformen denkbar. Insbesondere kann auf die obenstehend aufgeführten Anordnungen, die im Rahmen von (R1), (R2) und (Z1) beschrieben sind, verwiesen werden. Auch was die Bündelung oder das Aufteilen von Strömen aus den Reaktionsstufen (R2), (R3) und so weiter oder aus den Zwischenbehandlungen (Z2) und so weiter anbelangt, sind sämtliche geeigneten Ausführungsformen denkbar und es kann insbesondere auf die obenstehend aufgeführten Ausführungsformen, die im Rahmen von (R1), (R2) und (Z1) beschrieben sind, verwiesen werden.

Die Umsetzung der organischen Verbindung mit dem Hydroperoxid in der Stufe (R1) findet, wie vorstehend ausführlich beschrieben, in mindestens einem dafür geeigneten Reaktor statt. Als Edukte der Umsetzung werden die umzusetzende organische Verbindung, das Hydroperoxid und, sofern erforderlich, ein oder auch mehrere bei der Umsetzung geeignete und/oder erforderliche Lösungsmittel eingesetzt.

Bevorzugt wird im erfindungsgemäßen Verfahren jeweils ein Eduktstrom in den mindestens einen Reaktor aus (R1) geleitet, der Hydroperoxid, umzusetzende organische Verbindung und mindestens ein geeignetes Lösungsmittel umfaßt. Dabei ist ein Strom bevorzugt, bei dem die Konzentrationen der einzelnen Komponenten des Stroms so gewählt sind, daß der Strom flüssig und einphasig ist.

Gegebenenfalls kann dem Eduktstrom vor dem Einleiten in einen Reaktor aus (R1) mindestens eine basische, eine saure oder eine neutrale Verbindung oder ein Gemisch aus zwei oder mehr dieser Verbindungen zugegeben werden. Bevorzugt wird beispielsweise eine basische Verbindung zugegeben. Als basische Verbindungen sind beispielsweise solche zu nennen, die untenstehend im Rahmen der Basenzugabe bei mindestens einer Zwischenbehandlung aufgeführt sind.

Als Lösungsmittel können prinzipiell alle für die jeweilige Umsetzung geeigneten Lösungsmittel eingesetzt werden. Unter anderem bevorzugt sind beispielsweise
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen.

Als Lösungsmittel wird bei der Umsetzung von Hydroperoxid mit organischer Verbindung bevorzugt Methanol eingesetzt.

Bevorzugt werden im Eduktstrom oder in den Eduktstströmen dabei Hydroperoxidkonzentrationen verwendet, die im Bereich von 0,01 bis 10, besonders bevorzugt im Bereich von 0,1 bis 9 Gew.-%, weiter besonders bevorzugt im Bereich von 1 bis 8 Gew.-% und insbesondere im Bereich von 5 bis 7 Gew.-% liegen.

Je nach Temperatur, die zur Umsetzung der organischen Verbindung mit dem Hydroperoxid in (R1) gewählt wird, kann es im erfindungsgemäßen Verfahren sinnvoll sein, den Eduktstrom oder die Eduktströme vor dem Eingang in den Reaktor aus (R1) vorzutemperieren.

Die aus (R1) resultierende Mischung wird in mindestens einem Produktstrom (P1) der Zwischenbehandlung (Z1) zugeführt.

Während, wie oben beschrieben, sämtliche geeignete Zwischenbehandlungen denkbar sind, werden im Rahmen des erfindungsgemäßen Verfahrens als Zwischenbehandlungsstufen bevorzugt Verfahren eingesetzt, in denen aus einem Produktstrom, resultierend aus der Umsetzung von organischer Verbindung mit Hydroperoxid, nicht-umgesetztes Hydroperoxid abgetrennt wird oder zu einem Produktstrom Base zugegeben wird.

Demgemäß betrifft die vorliegende Erfindung auch ein Verfahren, wie oben beschrieben,
dadurch gekennzeichnet, daß mindestens eine Zwischenbehandlung
- eine destillative Abtrennung des Hydroperoxides aus mindestens einem Produktstrom oder
- eine Zugabe einer Base zu einem Produktstrom
ist.

Was die Zugabe von Base anbelangt, so sind hierbei prinzipiell sämtliche geeigneten Verfahrensführungen denkbar. So kann die Base beispielsweise vor der Zugabe vortemperiert werden. Ebenso kann die Base mit dem Produktstrom bei der Zugabe in geeigneter Weise vermischt werden.

Als Basen können sämtliche geeigneten basischen Verbindungen zugesetzt werden. Insbesondere bevorzugt werden basische Verbindungen eingesetzt, die im Rahmen des erfindungsgemäßen Verfahrens in gewünschter Weise die Umsetzung von Hydroperoxid mit organischer Verbindung beeinflussen. Werden, wie untenstehend ausführlich diskutiert, beispielsweise Zeolithe als heterogene Katalysatoren eingesetzt, so werden bevorzugt basische Verbindungen eingesetzt, die die Acidität dieser Zeolithe erniedrigen.

Unter anderem sind etwa Ammoniumsalze, Alkalisalze, wobei vor allem Lithium-, Natrium- und Kaliumsalze zu nennen sind, sowie Erdalkalisalze denkbar- Die Anionen dieser Salze umfassen beispielsweise Halogenide wie beispielsweise Chlorid und Bromid, Nitrat, Sulfat oder Hydroxid sowie die Anionen von Phosphor, Arsen, Antimon und Zinn enthaltenden Säuren wie z.B. Perchlorat, Phosphat, Hydrogenphosphat, Dihydrogenphophat, Arsenat und Stannat. Auch andere Anionen wie beispielsweise Formiat, Acetat, Hydrogencarbonat oder Carbonat sind denkbar.

Insbesondere sind hier etwa starke und schwache Basen zu nennen. Als starke Basen sind unter anderem etwa Natriumhydroxid oder Kaliumhydroxid möglich. Als schwache Basen sind beispielsweise etwa Ammoniumhydroxid, Carbonate, Hydrogencarbonate sowie hydrogenphosphate von Lithium, Natrium und Kalium wie etwa Ammoniumhydroxid, Natriumcarbonat, Natriumhydrogencarbonat, Kaliumcarbonat, Lithiumcarbonat, Kaliumhydrogencarbonat, Lithiumhydrogencarbonat, Kaliumhydrogenphosphat, Salze von Carbonsäuren mit 1 bis 10 Kohlenstoffatomen wie etwa Alkalimetallsalze oder Erdalkalimetallsalze oder Alkoholate von Alkoholen mit 1 bis 10 Kohlenstoffatomen wie etwa Erdalkalimetallalkoholate zu nennen.

Die Menge an Base, die zugegeben wird, kann im Rahmen des erfindungsgemäßen Vefahrens im wesentlichen beliebig gewählt werden und den jeweiligen Erfordernissen angepaßt werden. Bevorzugt wird soviel Base zugegeben, daß pro Mol Hydroperoxid höchstens 10 mmol Base, weiter bevorzugt höchstens 5 mmol Base und besonders bevorzugt weniger als 1 mmol Base vorliegen.

Ebenfalls bevorzugt wird im Rahmen einer Zwischenbehandlung Hydroperoxid aus einem Produktstrom abgetrennt, wobei dieses Hydroperoxid nicht-umgesetztes Hydroperoxid aus der vorhergehenden Reaktionsstufe ist. Weiter können im Rahmen einer Zwischenbehandlung auch zwei oder mehr Abtrennstufen vorgesehen werden, wobei diese parallel oder/und seriell angeordnet sein können. Bei serieller Anordnung ist es beispielsweise denkbar, in einer ersten Abtrennstufe nicht-umgesetztes Hydroperoxid aus der vorhergehenden Reaktionsstufe aus dem aus dieser Reaktionsstufe resultierenden Produktstrom weitgehend abzutrennen, und aus demjenigen Gemisch, das nach der weitgehenden Abtrennung des Hydroperoxids resultiert und noch Reste an Hydroperoxid umfaßt, in mindestens einer weiteren Abtrennstufe nochmals Hydroperoxid abzutrennen.

Die Abtrennung des Hydroperoxids kann im Rahmen des erfindungsgemäßen Verfahrens in einer oder auch mehrerer Zwischenbehandlungen erfolgen und kann weiter nach allen gängigen Verfahren gemäß dem Stand der Technik durchgeführt werden. Dabei können in unterschiedlichen Abtrennstufen auch unterschiedliche Abtrennmethoden eingesetzt werden. Im Rahmen einer Zwischenbehandlung können auch, wie bereits erwähnt, mehrere Abtrennstufen vorgesehen werden

Vorzugsweise erfolgt die Abtrennung des Hydroperoxides destillativ. Je nach den Anforderungen des Verfahrens ist dabei eine Abtrennung in einer oder mehreren Destillationskolonnen möglich. Vorzugsweise wird in einer Abtrennstufe zur Abtrennung des Hydroperoxides eine Destillationskolonne verwendet.

Im Rahmen des erfindungsgemäßen Verfahrens ist es denkbar, für jede Stufe, in der das Hydroperoxid abgetrennt wird, eine eigene Abtrennvorrichtung vorzusehen. Ebenso ist es möglich, bei entsprechender Reaktionsführung und bei mehreren Abtrennstufen die Abtrennungen in einer einzigen Abtrennvorrichtung durchzuführen.

Sind mehrere Abtrennstufen vorgesehen, ist es auch möglich, durch geeignete Reaktionsführung jeweils zwei oder auch mehr Abtrennstufen in jeweils einer Abtrennvorrichtung durchzuführen. Ganz allgemein ist es demgemäß möglich, für n Abtrennstufen insgesamt m Abtrennvorrichtungen vorzusehen, wobei 1 ≤ m ≤ n.

Sollte im Anschluß an die letzte Stufe, in der eine Umsetzung der organischen Verbindung mit dem Hydroperoxid stattfindet, noch eine weitere Abtrennung des Hydroperoxids gewünscht sein, beispielsweise, um eventuell restliches Hydroperoxid zu recyclen, so ist dies im Rahmen des erfindungsgemäßen Verfahrens selbstverständlich ebenfalls möglich.

Aus der Mischung, die aus einer Reaktionsstufe, in der die organische Verbindung mit dem Hydroperoxid umgesetzt wird, resultiert, können im erfindungsgemäßen Verfahren in einer oder mehreren Abtrennvorrichtungen neben dem Hydroperoxid auch die umgesetzte organische oder/und die nicht-umgesetzte organische Verbindung abgetrennt werden. Natürlich ist es auch möglich, nach Abtrennung des Hydroperoxids das verbleibende Reaktionsgut in eine weitere, speziell zu diesem Zweck vorgesehene Abtrennvorrichtung zu überführen und dort aus dem Reaktionsgut die umgesetzte oder/und die nicht-umgesetzte organische Verbindung abzutrennen.

In beiden Fällen ist es beispielsweise möglich, die umgesetzte oder/und die nicht-umgesetzte organische Verbindung in den n Abtrennvorrichtungen zu sammeln und nach Beendigung der Umsetzungen der organischen Verbindung mit dem Hydroperoxid abzutrennen.

Bevorzugt wird die umgesetzte organische Verbindung abgetrennt und wiederum bevorzugt in der jeweiligen Abtrennvorrichtung neben dem Hydroperoxid abgetrennt. Bei einer destillativen Abtrennung ist es beispielsweise möglich, die umgesetzte organische Verbindung über Kopf der Mischung zu entnehmen, und im Seitenabzug das Hydroperoxid aus der Mischung abzutrennen.

Im erfindungsgemäßen Verfahren ist es natürlich ebenfalls möglich, bei Verwendung einer Destillationsanlage als Abtrenneinrichtung das Hydroperoxid nicht über Seitenabzug, sondern über Sumpf aus der Mischung abzutrennen.

Erfolgt die Abtrennung des Hydroperoxids und/oder der umgesetzten organischen Verbindung in einer Destillationsanlage, ist es im erfindungsgemäßen Verfahren möglich, eventuell anfallende hochsiedende Komponenten der Mischung, die als Nebenprodukte aus der Umsetzung der organischen Verbindung mit dem Hydroperoxid anfallen, über Sumpf abzutrennen. Dabei ist es auch denkbar, beispielsweise durch Zugabe von vorzugsweise gasförmigen, niedrigsiedenden Komponenten, wie z.B. der organischen Verbindung, wie beispielsweise bevorzugt Propen, an sich, die Sumpftemperatur zu erniedrigen.

Beispiele für solche niedrigsiedenden Komponenten sind u.a. Kohlenwasserstoffe mit 1 bis 4 Kohlenstoffatomen wie beispielsweise Methan, Ethan, Propan, Butan, Ethen oder Butene. Ebenso können beispielsweise Stickstoff oder Argon eingesetzt werden.

Selbstverständlich ist es im erfindungsgemäßen Verfahren möglich, beispielsweise in (R1) oder/und (R2) oder in einer oder mehreren weiteren Reaktionsstufen, auch mehrere organische Verbindungen mit dem Hydroperoxid umzusetzen. Ebenso ist es denkbar, zur Umsetzung mehrere Hydroperoxide zu verwenden.

Werden mehrere organische Verbindungen und/oder mehrere Hydroperoxide miteinander in den jeweiligen Stufen umgesetzt, so können in den Mischungen bzw. Produktströmen, die aus den jeweiligen Reaktionsstufen resultieren, verschiedenartige Produkte, die aus den Umsetzungen resultieren, vorliegen. Werden diese wiederum in den jeweiligen Abtrennstufen destillativ abgetrennt, kann es notwendig sein, zur Abtrennung mehrere Destillationskolonnen vorzusehen. Ebenso kann die destillative Abtrennung mehrerer Hydroperoxide aus der Mischung mehrere Destillationskolonnen erforderlich machen.

Erfolgt bei destillativer Abtrennung hierbei auch die Abtrennung von umgesetzter organischer Verbindung, so wird die Destillation im allgemeinen so geführt, daß aus einem Produktstrom (P1) mindestens 50 %, bevorzugt mindestens 60 %, weiter bevorzugt mindestens 70 %, besonders bevorzugt mindestens 80 % und insbesondere bevorzugt mindestens 90 % der umgesetzten organischen Verbindung abgetrennt werden.

Bevorzugt wird die Abtrennung so geführt, daß eine flüssige Mischung, die das Hydroperoxid enthält, abgetrennt wird. Dabei ist es möglich, daß die abgetrennte Mischung, die das Hydroperoxid enthält, zusätzlich zum Hydroperoxid beispielsweise noch geringe Mengen an nicht umgesetzter organischer Verbindung und/oder umgesetzter organischer Verbindung enthält. Ebenso kann die Mischung, die das abgetrennte Hydroperoxid enthält, gegebenenfalls erforderliches Lösungsmittel enthalten.

Wird in der (Z1) auch die umgesetzte organische Verbindung abgetrennt, so resultiert aus dieser Abtrennung, aus der bevorzugt eine flüssige Mischung oder eine Flüssigkeit-Gas-Mischung erhalten wird, ein Strom, der neben der umgesetzten organischen Verbindung gegebenenfalls die nicht umgesetzte organische Verbindung und/oder geringe Mengen an Lösungsmittel enthält.

Wird, wie oben beschrieben, die Abtrennung in einer Destillationsanlage durchgeführt, und werden über Sumpf hochsiedende Anteile aus (P1) abgetrennt, so resultiert aus dieser Abtrennung ein Strom, der die hochsiedenden Anteile, die beispielsweise Nebenprodukte der Umsetzung in (R1) sein können, enthält.

Erfolgt im Rahmen der Zwischenbehandlung (Z1) eine wie vorstehend beschriebene destillative Auftrennung, so können verschiedene Produktströme (PZ1) der Stufe (R2) zugeführt werden, in der ebenfalls organische Verbindung mit Hydroperoxid umgesetzt wird.

Beispielsweise kann ein Produktstrom (PZ1), der abgetrenntes Hydroperoxid umfaßt, der Stufe (R2) zugeführt werden, in der dieses Hydroperoxid mit organischer Verbindung umgesetzt wird. Die hierbei eingesetzte mindestens eine organische Verbindung kann gleich oder verschieden von der organischen Verbindung sein, die in (R1) umgesetzt wurde. Bevorzugt wird in (R2) die gleiche organische Verbindung wie in (R1) eingesetzt. Weiter ist es denkbar, daß in (R2) der Produktstrom (PZ1), umfassend abgetrenntes Hydroperoxid, mit einem weiteren Produktstrom (PZ1), umfassend abgetrennte, nicht-umgesetzte organische Verbindung in Kontakt gebracht wird. Hierbei können zusätzlich zu diesen Strömen weiteres Hydroperoxid oder weitere organische Verbindung oder sowohl weiteres Hydroperoxid und weitere organische Verbindung in (R2) zugegeben werden. Die Begriffe "weiteres Hydroperoxid" beziehungsweise "weitere organische Verbindung" bezeichnen im Rahmen der vorliegenden Erfindung Hydroperoxid beziehungsweise organische Verbindung, die nicht in der Stufe (R1) eingesetzt wurden. Ebenso kann in (R2) ein Produktstrom (PZ1), umfassend abgetrenntes Hydroperoxid, ausschließlich mit weiterer organischer Verbindung in Kontakt gebracht und organische Verbindung mit Hydroperoxid umgesetzt werden. Weiter kann auch ein Produktstrom (PZ1), umfassend abgetrennte, nicht-umgesetzte organische Verbindung mit weiterem Hydroperoxid in Kontakt gebracht werden und organische Verbindung mit Hydroperoxid umgesetzt werden.

Wenn in der Zwischenbehandlung (Z1) Hydroperoxid aus (P1) abgetrennt wird, wird in einer besonders bevorzugten Verfahrensführung des erfindungsgemäßen Verfahrens mindestens ein Strom (PZ1). umfassend abgetrenntes, in (R1) nicht umgesetztes Hydroperoxid der Stufe (R2) zugeführt und in (R2) mit organischer Verbindung in Kontakt gebracht wird, die nicht aus (R1) resultiert und besonders bevorzugt frisch in das Verfahren eingebracht wird.

Wie obenstehend beschrieben ist es denkbar, im Rahmen der Zwischenbehandlung (Z1) verschiedene Verfahren anzuwenden. Demgemäß ist es beispielsweise möglich, in (Z1) zum einen mindestens eine Abtrenneinrichtung und zusätzlich mindestens eine Basenzugabe vorzusehen. Diesbezüglich kann beispielsweise aus dem Produktstrom (P1) zunächst in (P1) nicht-umgesetztes Hydroperoxid abgetrennt werden, woraus ein Produktstrom (PZ1), umfassend Hydroperoxid, resultiert, diesem Produktstrom (PZ1) Base zugegeben werden und der daraus resultierende neue Produktstrom (PZ1), umfassend Hydroperoxid und Base, der Reaktionsstufe (R2) zugeführt werden.

Wird in (R1) organische Verbindung mit Hydroperoxid umgesetzt, in (Z1) Hydroperoxid aus (P1), das in (R1) nicht umgesetzt wurde, unter Erhalt von (PZ1) abgetrennt, (PZ1) in (R2) eingeleitet und dort erneut organische Verbindung mit Hydroperoxid umgesetzt, werden die Reaktionsbedingungen in (R1) im erfindungsgemäßen Verfahren bevorzugt so gewählt, daß der Hydroperoxidumsatz im allgemeinen im Bereich von 70 bis 95 %, bevorzugt im Bereich von 80 bis 94,5 %, und insbesondere bevorzugt im Bereich von 85 bis 94 % liegt.

Weiter werden Druck, Temperatur und Verweilzeit des Reaktionsgutes in (R1) bevorzugt so gewählt, daß die Mischung, die aus der Umsetzung resultiert, flüssig und einphasig ist.

Dabei werden Drücke gewählt, die im allgemeinen im Bereich vom Eigendruck bis 100 bar liegen, bevorzugt im Bereich vom Eigendruck bis 40 bar und besonders bevorzugt im Bereich vom Eigendruck bis 30 bar.

Die Temperaturen in (R1) liegen im allgemeinen im Bereich von 0 bis 120 °C, bevorzugt im Bereich von 10 bis 100 °C, weiter bevorzugt im Bereich von 20 bis 90 °C und besonders bevorzugt im Bereich von 25 bis 80 °C.

Die Konzentration der umzusetzenden organischen Verbindung wird beispielsweise so gewählt, daß das molare Verhältnis von umzusetzender organischer Verbindung zu Hydroperoxid im Bereich von 0,7 bis 20, bevorzugt im Bereich von 0,8 bis 5,0, weiter besonders bevorzugt im Bereich von 0,9 bis 2,0 und insbesondere im Bereich von 1,0 bis 1,6 liegt.

In (R2) wird dann die Konzentration der umzusetzenden organischen Verbindung so gewählt, daß das molare Verhältnis von umzusetzender organischer Verbindung zu Hydroperoxid bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt.

Die Umsetzung in (R2) wird in diesem Fall bei einem Druck, einer Temperatur und einer Verweilzeit des Reaktionsgutes durchgeführt, so daß Hydroperoxidumsätze erzielt werden, die im allgemeinen im Bereich von ≥ 90%, bevorzugt im Bereich von ≥ 92%, weiter bevorzugt im Bereich ≥ 95% und besonders bevorzugt im Bereich von 95 bis 99,5% liegen.

Dabei werden Drücke gewählt, die im allgemeinen im Bereich vom Eigendruck bis 100 bar liegen, bevorzugt im Bereich vom Eigendruck bis 40 bar und besonders bevorzugt im Bereich vom Eigendruck bis 30 bar.

Die Temperaturen liegen im allgemeinen im Bereich von 0 bis 120 °C, bevorzugt im Bereich von 10 bis 100 °C, weiter bevorzugt im Bereich von 20 bis 90 °C und besonders bevorzugt im Bereich von 25 bis 80 °C.

Die Verweilzeiten des Reaktionsgutes in (R1) oder/und (R2) können im wesentlichen frei bestimmt werden und beispielsweise den gewünschten Umsätzen, wie obenstehend beschrieben, angepaßt werden. Im allgemeinen liegen sie bei weniger als 5 Stunden, bevorzugt bei weniger als 3 Stunden, weiter bevorzugt bei weniger als 1 Stunde und besonders bevorzugt bei etwa einer halben Stunde.

Wie bereits obenstehend beschrieben, kann das erfindungsgemäße Verfahren neben (Z1) mindestens eine weitere Zwischenbehandlung und neben (R1) und (R2) mindestens eine weitere Reaktionsstufe aufweisen. In einer besonders bevorzugten Ausführungsform umfaßt die vorliegende Erfindung Reaktionsstufen (R1) und (R2), von denen jede mindestens zwei parallel geschaltete Reaktoren aufweist, eine Zwischenbehandlung (Z1), in der Base zugegeben wird, eine Zwischenbehandlung (Z2), in der aus dem Produktstrom (P2) Hydroperoxid abgetrennt wird und eine Reaktionsstufe (R3).

Demgemäß betrifft die vorliegende Erfindung ein Verfahren, wie oben beschrieben, dadurch gekennzeichnet, daß
- in der Reaktionsstufe (R1) Hydroperoxid mit organischer Verbindung in mindestens zwei parallel geschalteten Reaktoren in Anwesenheit eines Katalysators unter Erhalt mindestens eines Produktstromes (P1), umfassend nicht umgesetztes Hydroperoxid, nicht umgesetzte organische Verbindung und umgesetzte organische Verbindung, umgesetzt wird,
- in der Zwischenbehandlung (Z1) zu dem mindestens einen Produktstrom (P1) Base unter Erhalt mindestens eines Produktstromes (PZ1) zugegeben wird,
- in der Reaktionsstufe (R2) nicht umgesetztes Hydroperoxid und nicht umgesetzte organische Verbindung, enthalten in dem mindestens einen Produktstrom (PZ1), unter Erhalt mindestens eines Produktstromes (P2), umfassend nicht umgesetztes Hydroperoxid, in mindestens zwei parallel geschalteten Reaktoren umgesetzt werden,
- in einer Zwischenbehandlung (Z2) aus dem mindestens einen Produktstrom (P2) nicht umgesetztes Hydroperoxid unter Erhalt mindestens eines Produktstromes (PZ2), umfassend Hydroperoxid, destillativ abgetrennt wird, und
- das Hydroperoxid, enthalten in dem mindestens einen Produktstrom (PZ2), in einer Reaktionsstufe (R3) mit organischer Verbindung unter Erhalt mindestens einen Produktstroms (P3) umgesetzt wird.

In dieser bevorzugten Ausführungsform des erfindungsgemäßen Verfahren werden in (R1), (R2) und (R3) Drücke gewählt, die im allgemeinen im Bereich vom Eigendruck bis 100 bar liegen, bevorzugt im Bereich vom Eigendruck bis 40 bar und besonders bevorzugt im Bereich vom Eigendruck bis 30 bar liegen. Die Drücke können hierbei in allen 3 Reaktionsstufen gleich sein oder sich voneinander unterscheiden.

Die Temperaturen in (R1), (R2) und (R3) liegen bei dieser bevorzugten Ausführungsform im allgemeinen im Bereich von 0 bis 120 °C, bevorzugt im Bereich von 10 bis 100 °C, weiter bevorzugt im Bereich von 20 bis 90 °C und besonders bevorzugt im Bereich von 25 bis 80 °C. Die Temperaturen können hierbei in allen 3 Reaktionsstufen gleich sein oder sich voneinander unterscheiden.

Im Rahmen dieser bevorzugten Ausführungsform wird die Konzentration der umzusetzenden organischen Verbindung in (R1), (R2) und (R3) so gewählt, daß das molare Verhältnis von umzusetzender organischer Verbindung zu Hydroperoxid bevorzugt im Bereich von 0,7 bis 20, weiter bevorzugt im Bereich von 0,8 bis 5,0, besonders bevorzugt im Bereich von 0,9 bis 2,0 und insbesondere im Bereich von 1,0 bis 1,6 liegt. Die jeweiligen molaren Verhältnisse können in (R1), (R2) und (R3) gleich oder verschieden voneinander sein.

Die Umsetzung in (R1) wird im Rahmen dieser bevorzugten Ausführungsform bei einem Druck. einer Temperatur und einer Verweilzeit des Reaktionsgutes durchgeführt. so daß Hydroperoxidumsätze erzielt werden, die im allgemeinen im Bereich von ≥ 50 %, bevorzugt im Bereich von ≥ 60 %, weiter bevorzugt im Bereich von ≥ 70 % und besonders bevorzugt im Bereich von 70 bis 80% liegen.

Die Umsetzung in (R2) wird im Rahmen dieser bevorzugten Ausführungsform bei einem Druck, einer Temperatur und einer Verweilzeit des Reaktionsgutes durchgeführt, so daß Hydroperoxidumsätze erzielt werden, die im allgemeinen im Bereich von ≥ 80 %, bevorzugt im Bereich von ≥ 85 % und besonders bevorzugt im Bereich von 87 bis 93 % liegen.

Die Umsetzung in (R3) wird im Rahmen dieser bevorzugten Ausführungsform bei einem Druck. einer Temperatur und einer Verweilzeit des Reaktionsgutes durchgeführt, so daß Hydroperoxidumsätze erzielt werden, die im allgemeinen im Bereich von ≥ 98 % und besonders bevorzugt im Bereich von 99 bis 99,99 % liegen.

In dieser bevorzugten Ausführungsform, in der in der Zwischenbehandlung (Z1) Base zugegeben wird und in der Zwischenbehandlung (Z2) Hydroperoxid abgetrennt wird, werden in Reaktionsstufe (R2) im wesentlichen die gleichen Reaktionsbedingungen wie in Reaktionsstufe (R1) gewählt. Als Reaktionsbedingungen, die in Stufe (R3) gewählt werden, sind diejenigen bevorzugt, die obenstehend detailliert bei der bevorzugten Ausführungsform beschrieben sind, in der in (R1) organische Verbindung mit Hydroperoxid umgesetzt wird, in (Z1) Hydroperoxid abgetrennt wird und in (R2) organische Verbindung mit Hydroperoxid umgesetzt wird.

Als Reaktoren können alle denkbaren, für die jeweiligen Reaktionen in den jeweiligen Reaktionsstufen am besten geeigneten Reaktoren eingesetzt werden. Dabei ist im erfindungsgemäßen Verfahren ein Reaktor nicht auf einen einzelnen Behälter beschränkt. Vielmehr ist es auch möglich, als Reaktor in (R1) oder (R2) oder einer weiteren Reaktionsstufe beispielsweise eine Rührkesselkaskade einzusetzen. Bevorzugt werden im erfindungsgemäßen Verfahren als Reaktoren Festbettreaktoren verwendet.

Demgemäß betrifft die vorliegende Erfindung auch ein Verfahren, wie oben beschrieben,
dadurch gekennzeichnet, daß als Reaktoren Festbettreaktoren, vorzugsweise isotherme Festbettreaktoren eingesetzt werden.

Insbesondere bevorzugt sind im erfindungsgemäßen Verfahren isotherme Festbettreaktoren, die bevorzugt eingesetzt werden, wenn in (R1) oder/und (R2) organische Verbindung mit Hydroperoxid umgesetzt wird. ohne daß in einer Zwischenbehandlung Hydroperoxid abgetrennt wird. Adiabatische Festbettreaktoren werden bevorzugt dann eingesetzt, wenn abgetrenntes, in (R1) nicht umgesetztes Hydroperoxid, beispielsweise in (R2) oder in (R3), mit organischer Verbindung umgesetzt wird.

Daher betrifft die vorliegende Erfindung auch eine Vorrichtung, umfassend mindestens zwei parallel geschaltete isotherme Festbettreaktoren, eine Abtrenneinrichtung und mindestens einen adiabatischen Festbettreaktor.

Insbesondere bevorzugt beschreibt die vorliegende Erfindung eine Vorrichtung, umfassend mindestens zwei parallel geschaltete isotherme Festbettreaktoren, mindestens zwei Vorrichtungen zur Zugabe von basischer Verbindung, mindestens zwei weitere parallel geschaltete isotherme Festbettreaktoren, mindestens eine Abtrenneinrichtung und mindestens einen adiabatischen Festbettreaktor.

Weiter bevorzugt werden als Festbettreaktoren Festbettrohrreaktoren eingesetzt.

Als Hydroperoxid können im erfindungsgemäßen Verfahren sämtliche aus dem Stand der Technik bekannten Hydroperoxide, die für die Umsetzung der organischen Verbindung geeignet sind, eingesetzt werden.

Beispiele für solche Hydroperoxide sind etwa tert-Butylhydroperoxid oder Ethylbenzolhydroperoxid, die im oben genannten SRI-Report 2E "Propylene Oxide" genannt sind. Das tert-Butylhydroperoxid wird hierin hergestellt ausgehend von Isobutan und Sauerstoff. Das Ethylbenzolhydroperoxid wird hergestellt ausgehend von Ethylbenzol und Sauerstoff.

Bevorzugt wird als Hydroperoxid im vorliegenden Verfahren Wasserstoffperoxid eingesetzt. Demgemäß beschreibt die vorliegende Erfindung auch ein Verfahren, wie oben beschrieben, das dadurch gekennzeichnet ist, daß als Hydroperoxid Wasserstoffperoxid verwendet wird. Bevorzugt wird dabei eine wäßrige Wasserstoffperoxidlösung verwendet.

Zur Herstellung von Wasserstoffperoxid kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmanns Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus Wasserstoff und Sauerstoff.

Vor dem Einsatz von Wasserstoffperoxid im erfindungsgemäßen Verfahren ist es möglich, beispielsweise eine kommerziell erhältliche Wasserstoffperoxid-Lösung von unerwünschten Ionen zu befreien. Dabei sind unter anderem Methoden denkbar, wie sie beispielsweise in der WO 98/54086, in der DE-A 42 22 109 oder in der WO 92/06918 beschrieben sind. Ebenso kann mindestens ein Salz, das in der Wasserstoffperoxid-Lösung enthalten ist, durch eine Vorrichtung aus der Wasserstoffperoxid-Lösung mittels Ionenaustausch entfert werden, die dadurch gekennzeichnet ist, daß die Vorrichtung mindestens ein nicht-saures lonenaustauscherbett mit einer Strömungsquerschnittsfläche F und einer Höhe H aufweist, wobei die Höhe H des lonenaustauscherbettes kleiner oder gleich 2,5 • F^{1/2} und insbesondere kleiner oder gleich 1,5 • F^{1/2} ist. Im Rahmen der vorliegenden Erfindung können prinzipiell alle nicht-sauren Ionenaustauscherbetten mit Kationenaustauscher und/oder Anionenaustauscher eingesetzt werden. Auch innerhalb eines Ionenaustauscherbettes können Kationen- und Anionenaustauscher als sogenannte Mischbetten verwendet werden. In einer bevorzugten Ausführungsform der vorliegenden Erfindung wird nur ein Typ von nicht-saueren Ionenaustauschern eingesetzt. Weiter bevorzugt ist der Einsatz eines basischen Ionentauschers, besonders bevorzugt der eines basischen Anionentauschers und weiter besonders bevorzugt der eines schwach basischen Anionentauschers.

Als Katalysatoren zur Umsetzung der organischen Verbindung mit Hydroperoxid sind prinzipiell alle, bevorzugt alle heterogenen Katalysatoren denkbar, die für die jeweilige Umsetzung geeignet sind. Bevorzugt werden dabei Katalysatoren verwendet, die ein poröses oxidisches Material wie z.B. einen Zeolith umfassen. Vorzugsweise werden Katalysatoren eingesetzt, die als poröses oxidisches Material ein Titan-, Vanadium-, Chrom-, Niob- oder Zirkonium-haltigen Zeolith umfassen.

Im besonderen existieren Zeolithe, die kein Aluminium enthalten und bei denen im Silikatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WEI-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-12 zu nennen. Ebenso seien die titanhaltigen Strukturen RUB-29, RUB-30, RUB-31 und RUB-23 genannt.

Als besonders bevorzugt sind für das erfindungsgemäße Verfahren Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Insbesondere bevorzugt wird im erfindungsgemäßen Verfahren ein heterogener Katalysator, der das titanhaltige Silikalit TS-1 umfaßt, verwendet.

Dabei ist es im erfindungsgemäßen Verfahren möglich, als Katalysator das poröse oxidische Material an sich zu verwenden. Selbstverständlich ist es jedoch auch möglich, als Katalysator einen Formkörper einzusetzen, der das poröse oxidische Material umfaßt. Dabei können zur Herstellung des Formkörpers, ausgehend von dem porösen oxidischen Material, alle Verfahren gemäß dem Stand der Technik eingesetzt werden.

Unter den Umsetzungen, die im erfindungsgemäßen Verfahren möglich sind, seien beispielhaft die folgenden genannt:
die Epoxidation von Olefinen wie z.B. die Herstellung von Propenoxid aus Propen und H₂O₂ oder aus Propen und Gemischen, die H₂O₂ in situ liefern;
Hydroxylierungen wie z.B die Hydroxylierung mono-, bi- oder polycyclischer Aromaten zu mono-, di- oder höher substituierten Hydroxyaromaten, beispielsweise die Umsetzung von Phenol und H₂O₂ oder von Phenol und Gemischen, die H₂O₂ in situ liefern, zu Hydrochinon;
die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
die Baeyer-Villiger-Oxidation.

Bevorzugt werden im erfindungsgemäßen Verfahren organische Verbindungen umgesetzt, die mindestens eine C-C-Doppelbindung aufweisen. Demgemäß beschreibt die vorliegende Erfindung ein Verfahren, wie oben beschrieben, das dadurch gekennzeichnet ist, daß die organische Verbindung mindestens eine C-C-Doppelbindung aufweist.

Als Beispiele für solche organischen Verbindungen mit mindestens einer C-C-Doppelbindung seien folgende Alkene genannt:

Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbomen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden im erfindungsgemäßen Verfahren Alkene verwendet, die 2 bis 8 Kohlenstoffatome enthalten. Besonders bevorzugt werden Ethen, Propen, und Buten umgesetzt. Insbesondere bevorzugt wird Propen umgesetzt.

Demgemäß betrifft die vorliegende Erfindung auch ein Verfahren, wie oben beschrieben, dadurch gekennzeichnet, daß Wasserstoffperoxid mit Alken, vorzugsweise Propen, zu Alkenoxid, vorzugsweise Propylenoxid, umgesetzt wird, wobei als Katalysator ein Zeolith-Katalysator, vorzugsweise ein Titan-Silikalit-Katalysator, insbesondere ein Titansilikalit-Katalysator der Struktur TS-1, eingesetzt wird.

Als Lösungsmittel, einsetzbar bei der Umsetzung von Wasserstoffperoxid mit Alken, vorzugsweise Propen, zu Alkenoxid, vorzugsweise Propylenoxid eignen sich prinzipiell alle dem Fachmann dafür bekannten Lösungsmittel. Bevorzugt werden organische Lösungsmittel, wie Alkohole, einzeln oder als Gemisch aus zwei oder mehr davon, eingesetzt. Auch Alkohol/Wasser Mischungen sind einsetzbar. Bevorzugt wird im Rahmen des Verfahrens als Lösungsmittel Methanol verwendet.

Die dabei einsetzbaren Lösungsmittelmengen sind in breiten Grenzen variierbar. Mögliche Lösungsmittelmengen im Rahmen des Verfahrens liegen zwischen 5 und 25 g Methanol pro Gramm eingesetztem Wasserstoffperoxid. Bevorzugt wird das Lösungsmittel in Mengenbereichen von 8 bis 16 g Methanol pro Gramm eingesetztem Wasserstoffperoxid, besonders bevorzugt von 10 bis 14 g Methanol pro Gramm eingesetztem Wasserstoffperoxid eingesetzt.

Wie obenstehend beschrieben wird mindestens ein Katalysator, der in mindestens einem der parallel geschalteten Reaktoren enthalten ist, regeneriert, wobei bevorzugt so gearbeitet wird, daß das kontinuierliche Verfahren nicht unterbrochen wird. Die Abkopplung des mindestens eine Reaktors, in dem sich der zu regenerierende Katalysator befindet, aus dem Verfahrensstrang kann hierbei nach sämtlichen geeigneten Methoden erfolgen.

Nach der Abkopplung des Reaktors aus dem Verfahrensstrang kann der im Reaktor enthaltene Katalysator nach sämtlichen geeigneten Verfahren aus dem Stand der Technik regeneriert werden. Insbesondere ist es möglich, den Katalysator aus dem Reaktor auszubauen, zu regenerieren und wieder einzubauen. Bevorzugt wird der Katalysator im Reaktor regeneriert. Je nach Art des Katalysators kann die Regenerierungsmethode gewählt werden. Für die Zeolith-Katalysatoren, die im Rahmen des besonders bevorzugten Verfahrens eingesetzt werden, sind beispielsweise folgende Möglichkeiten der Regenerierung zu nennen:
1. ein Verfahren, das das Erhitzen eines verbrauchten Katalysators bei einer Temperatur von weniger als 400 °C, aber höher als 150 °C in Gegenwart von molekularem Sauerstoff für einen Zeitraum, der ausreichend zur Erhöhung der Aktivität des verbrauchten Katalysators ist, umfaßt, wie es in der EP-A 0 743 094 beschrieben ist;
2. ein Verfahren, das das Erwärmen eines verbrauchten Katalysators bei einer Temperatur von 150 °C bis 700 °C in Gegenwart eines Gasstroms, der höchstens 5 Vol.-% molekularen Sauerstoffs enthält, über einen Zeitraum, der ausreicht, um die Aktivität des verbrauchten Katalysators zu verbessern, umfaßt, wie es in der EP-A 0 790 075 beschrieben ist;
3. ein Verfahren, in dem ein verbrauchter Katalysator durch Erhitzen bei 400 bis 500 °C in Gegenwart eines Sauerstoff enthaltenden Gases oder durch Waschen mit einem Lösungsmittel, vorzugsweise bei einer Temperatur, die 5 °C bis 150 °C höher ist als die während der Umsetzung verwendete Temperatur, behandelt wird, wie es in der JP-A 3 11 45 36 beschrieben wird;
4. ein Verfahren, in dem ein verbrauchter Katalysators durch Calcinieren bei 550 °C an Luft oder durch Waschen mit Lösungsmitteln behandelt wird, wobei die Aktivität des Katalysators wiederhergestellt wird, wie es in "Proc. 7^{th} Intern. Zeolite Conf. 1986 (Tokyo)" beschrieben wird;
5. ein Verfahren zur Regenerierung eines Katalysators, das die folgenden Stufen (A) und (B) umfaßt:
   (A) Aufheizen eines zumindest teilweise deaktivierten Katalysators auf eine Temperatur im Bereich 250 °C bis 600 °C in einer Atmosphäre, die weniger als 2 Vol.-% Sauerstoff enthält, und
   (B) Beaufschlagen des Katalysators bei einer Temperatur im Bereich von 250 bis 800 °C, vorzugsweise 350 bis 600 °C, mit einem Gasstrom, der einen Gehalt an einer Sauerstoff liefernden Substanz oder an Sauerstoff oder an einem Gemisch aus zwei oder mehr davon im Bereich von 0,1 bis 4 Vol.-% aufweist,
      wobei das Verfahren auch die weiteren Stufen (C) und (D),
   (C) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von mehr als 4 bis 100 Vol.-% aufweist,
   (D) Abkühlen des in Stufe (C) erhaltenen regenerierten Katalysators in einem Inertgasstrom, der bis zu 20 Vol.-% eines Flüssigkeitsdampfes, ausgewählt aus der Gruppe bestehend aus Wasser, einem Alkohol, einem Aldehyd, einem Keton, einem Ether, einer Säure, einem Ester, einem Nitril, einem Kohlenwasserstoff sowie einem Gemisch aus zwei oder mehr davon,
   umfassen kann. Details bezüglich dieses Verfahrens sind der DE-A 197 23 949.8 zu entnehmen;
6. ein Verfahren, in dem ein verbrauchter Katalysator durch thermische Behandlung unter einem Gasstrom bei Temperaturen von mindestens 130 °C derart regeneriert wird, daß die Zeit, in der sich der Gasstrom über dem Katalysator befindet, 2 Stunden nicht übersteigt. Details bezüglich dieses Verfahrens lassen sich der WO 98/18556 entnehmen.

Weiter ist es ebenso denkbar, den Katalysator zur Regenerierung mit mindestens einer Wasserstoffperoxidlösung oder auch mit einer oder mehreren oxidierenden Verbindungen zu waschen. Selbstverständlich können auch die vorstehend beschriebenen Methoden in geeigneter Weise miteinander kombiniert werden.

Während der Umsetzungen der organischen Verbindung mit Hydroperoxid können weiterhin der pH-Wert oder/und die Temperatur des Reaktionsmediums oder/und der Druck, unter dem die Umsetzung erfolgt, geändert werden.

Bevorzugt wird hierbei der pH-Wert des Reaktionsmediums durch Änderung des pH-Wertes der Hydroperoxidlösung, die dem Reaktionsmedium zugegeben wird, erreicht. Dies ist hinsichtlich (R1) beispielsweise durch geeignete Behandlung eines Eduktstromes, der Hydroperoxidlösung enthält, möglich. Wird beispielsweise in (Z1) oder/und (Z2) Hydroperoxidlösung abgetrennt, so kann derjenige Strom, der abgetrenntes Hydroperoxid umfaßt, zur Änderung des pH-Wertes in geeigneter Weise behandelt werden.

Der pH-Wert eines Hydroperoxidstromes, insbesondere der Wasserstoffperoxidlösung, kann prinzipiell nach allen gängigen Verfahren eingestellt werden. Dabei ist lediglich darauf zu achten, daß bei Zugabe von sauren oder basischen Verbindungen oder bei Zugabe einer Lösung, die saure oder basische Verbindungen umfaßt, zur Hydroperoxidlösung die nachfolgende Umsetzung der organischen Verbindung mit Hydroperoxid nicht nachteilig beeinflußt wird. Insbesondere wird der pH-Wert der Hydroperoxidlösung
(a) durch Behandlung der Hydroperoxidlösung mit mindestens einem lonentauscher oder
(b) durch Zugabe
   (aa) einer sauren Verbindung oder
   (bb) einer basischen Verbindung oder
   (cc) einer neutralen Verbindung oder
   (dd) eines Gemisches aus zwei oder mehr davon
   zur Hydroperoxidlösung oder
(c) durch eine Kombination der Methoden (a) und (b)
geändert.

Hierbei sind prinzipiell sowohl stark basische als auch schwach basische Verbindungen oder sowohl stark saure als auch schwach saure Verbindungen geeignet. Insbesondere sind unter anderem die folgenden Salze denkbar:

Ammoniumsalze, Alkalisalze, wobei vor allem Lithium-, Natrium- und Kaliumsalze zu nennen sind, sowie Erdalkalisalze. Die Anionen dieser Salze umfassen beispielsweise Halogenide wie beispielsweise Chlorid und Bromid, Nitrat, Sulfat oder Hydroxid sowie die Anionen von Phosphor, Arsen, Antimon und Zinn enthaltenden Säuren wie z.B. Perchlorat, Phosphat, Hydrogenphosphat, Dihydrogenphophat, Arsenat und Stannat. Auch andere Anionen wie beispielsweise Formiat, Acetat, Hydrogencarbonat oder Carbonat sind denkbar.

Als Beispiele seien unter anderem Lithiumchlorid, Lithiumbromid, Natriumbromid, Lithiumnitrat, Natriumnitrat, Kaliumnitrat, Lithiumsulfat, Natriumsulfat, Kaliumsulfat, Natriumhydroxid, Kaliumhydroxid, Ammoniumhydroxid, Natriumcarbonat, Natriumhydrogencarbonat, Kaliumcarbonat, Lithiumcarbonat, Kaliumhydrogencarbonat, Lithiumhydrogencarbonat und Kaliumhydrogenphosphat sowie Lithium-, Magnesium-, Calcium-, Barium oder Ammoniumacetat. Ebenfalls zu nennen sind Carboxylate von Carbonsäuren, insbesondere von Carbonsäuren mit 1 bis 10 Kohlenstoffatomen, sowie Alkoholate von Alkoholen mit 1 bis 10 Kohlenstoffatomen. Weitere Beispiele sind unter anderem Ammoniumdihydrogenphosphat, Natriumdihydrogenphosphat, Kaliumdihydrogenphosphat, Dinatriumdihydrogenpyrophsphat.

Als Ionentauscher lassen sich hierbei grundsätzlich alle dem Fachmann bekannten Ionenaustauscher einsetzen, beispielsweise organische Ionenaustauscher, etwa auf Polystyrolbasis, oder anorganische lonenaustauscher, etwa Hydrotalcite sowie andere Schichtsilikate, die austauschbare Carbonat-, Hydrogencarbonat- oder Hydroxidgruppen enthalten können.

Beispiele für die im Rahmen der vorliegenden Erfindung besonders bevorzugten basischen Ionenaustauscher sind Polystyrolharze mit tertiären Amingruppen, etwa die kommerziell erhältlichen Anionenaustauscher Lewatit® MP62 und Lewatit® MP 63 sowie Dowex® MWA/1 und Dowex® AMW-500. Darüber hinaus ist auch die Verwendung von etwa quartäre Ammoniumgruppen enthaltenden Polystyrolharzen mit Hydroxid-Gegenionen denkbar. Beispielhaft seien hierbei die kommerziell erhältlichen Austauscher Lewatit® OC-1950 sowie Dowex® 1, Dowex® 2, Dowex® 11, Dowex® 21K und Dowex® 550A genannt.

Was die Änderung der Temperatur des Reaktionsmediums, in dem die Umsetzung des Hydroperoxids mit der organischen Verbindung erfolgt, anbelangt, so sind im wesentlichen sämtliche denkbaren Verfahren möglich. Beispielsweise kann die Temperatur des Reaktionsmediums über die Temperatur mindestens eines Eduktstromes gesteuert werden, der dem Reaktionsmedium zugeführt wird.

Bevorzugt wird die Temperatur über eine geeignete Thermostatisierung des mindestens einen Reaktors variiert. Auch hierbei sind sämtlichen geeigneten Methoden anwendbar. Beispielsweise kann der mindestens eine Reaktor mit einem Doppelmantel versehen sein, durch den beispielsweise eine Flüssigkeit geleitet wird, über deren Temperatur die Temperatur des Reaktionsmediums im Reaktor eingestellt wird. Hierbei ist es selbstverständlich auch denkbar, verschiedene Zonen des mindestens einen Reaktors mit voneinander separierten Doppelmänteln zu versehen und so die verschiedenen Zonen mit beispielsweise Flüssigkeiten unterschiedlicher Temperatur zu umspülen. Die zonenweise unterschiedliche Temperierung ist selbstverständlich nicht auf Anordnungen beschränkt, bei denen der Reaktor mit einem oder mehreren Doppelmänteln versehen ist, sondern auch durch alle anderen geeigneten Methoden erreichbar.

In einer bevorzugten Ausführungsform des erfindungsgemäßen Verfahrens resultiert nach Abtrennung des Hydroperoxids und erneuter Umsetzung des abgetrennten Hydroperoxids mit Alken ein Gemisch als Rohaustrag, aus dem bevorzugt nicht-umgesetztes Alken abgetrennt und in das Verfahren als Edukt rückgeführt wird. In einer möglichen Ausführungsform wird hierbei dieser Rohaustrag einer Destillation unterworfen, wonach das Rohprodukt in eine Leichtsiederfraktion, die Alken und Verbindungen mit einem niedrigeren Siedepunkt als Alken enthält, und eine Schwersiederfraktion, die das Alkenoxid und Verbindungen mit einem höheren Siedepunkt als Alken enthält, aufgetrennt wird. In dieser Leichtsiederfraktion sammelt sich unter anderem Sauerstoff in einer Konzentration an, die die Leichtsiederfraktion zu einem zündfähigen Gemisch macht.

Um Alken gefahrlos abdestillieren zu können, ist es möglich, bei der destillativen Abtrennung von Alken aus dem Leichtsiedergemisch im oberen Teil der Trenneinrichtung einen inerten Stoff mit einem Siedepunkt, der niedriger als der von Alken, bevorzugt Propen, ist, bevorzugt Methan, in einer solchen Menge zuzugeben, daß der Sauerstoff bis zu einer Konzentration verdünnt ist, bei der das Gemisch nicht mehr zündfähig ist. Ebenso ist es möglich, ein Verfahren zur Aufarbeitung eines Gemisches (G1), umfassend ein Alken und Sauerstoff einzusetzen, in dem
(i) Sauerstoff nicht-destillativ aus dem Gemisch (G1) unter Erhalt eines Gemisches (G2) entfernt wird und
(ii) aus dem Gemisch (G2) das Alken destillativ abgetrennt wird.

Bevorzugt wird hierbei im erfindungsgemäßen Verfahren Sauerstoff durch Verbrennung entfernt. Ebenso bevorzugt wird eine Ausführungsform, gemäß der das Gemisch (G1) Reaktionsbedingungen unterworfen wird, bei denen der im Gemisch enthaltene Sauerstoff mit einer geeigneten chemischen Verbindung reagiert. In einer bevorzugten Ausführungsform des erfindungsgemäßen Verfahrens erfolgt die Verbrennung des Sauerstoffs, der im Gemisch (G1) enthalten ist, unter Einsatz mindestens eines geeigneten Katalysators. Hierfür sind sämtliche geeigneten Katalysatoren oder Katalysatorgemische einsetzbar. Bevorzugt werden unter anderem Edelmetalle wie beispielsweise Pt, Rh oder Pd, die auf geeigneten Trägern wie beispielsweise Metalloxiden aufgebracht sein können. Beispielsweise werden auf Al₂O₃ geträgerte Pd-Katalysatoren eingesetzt. Ebenso sind auch Kupferchromit-Katalysatoren zu nennen. Unter anderem seien hier etwa die kommerziell erhältlichen Katalysatoren R0-25/50 S6, R0-20/47 K2-4 beziehungsweise R3-20 S6 der Firma BASF AG zu nennen.

In vielen Fällen umfaßt der Rohaustrag aus der Epoxidierung noch zu Alken korrespondierendes Alkan. In diesem Fall ist es möglich, aus einem als Rohaustrag der Epoxidation anfallenden Gemisch (G1), umfassend nicht-umgesetztes Sauerstoff, Alken und Alkan, den Sauerstoff durch Oxydehydrierung des Alkans unter Erhalt des Alkens aus dem Gemisch (G1) zu entfernen. Die Oxydehydrierung des mindestens einen Alkans kann sowohl katalytisch oder auch nicht-katalytisch erfolgen. Bevorzugt wird die Oxydehydrierung unter Verwendung eines geeigneten Katalysators durchgeführt. Hinsichtlich dieser Katalysatoren sei unter anderem beispielsweise auf M. Xu, J. H. Lunsford, React. Kinet. Catal. Lett. 57 (1996) S. 3-11 und auf B. Delmon, Stud. Surf. Sci. Catal. 110 (1997) S. 43-59 und die darin zitierten Literaturstellen verwiesen, die diesbezüglich vollumfänglich in den Kontext der vorliegenden Anmeldung durch Bezugnahme einbezogen werden.

## Patentansprüche

1. Kontinuierliches Verfahren zur Umsetzung einer organischen Verbindung mit Hydroperoxid in Gegenwart eines Katalysators, wobei
- die organische Verbindung in einer Reaktionsstufe (R1) mit Hydroperoxid in Anwesenheit eines Katalysators unter Erhalt mindestens eines Produktstroms (P1) umgesetzt wird,
- der mindestens eine Produktstrom (P1) einer Zwischenbehandlung (Z1) zugeführt wird, wobei aus (Z1) mindestens ein Produktstrom (PZ1), umfassend Hydroperoxid, erhalten wird, und
- der mindestens eine Produktstrom (PZ1) einer Reaktionsstufe (R2) zugeführt wird, in der Hydroperoxid mit der organischen Verbindung in Anwesenheit eines Katalysators unter Erhalt mindestens eines Produktstroms (P2) umgesetzt wird,
**dadurch gekennzeichnet, daß** in den Reaktionsstufen (R1) und (R2) jeweils eine Reaktoranordnung, umfassend mindestens zwei parallel geschaltete Reaktoren, eingesetzt wird.

2. Verfahren nach Anspruch 1, **dadurch gekennzeichnet, daß** mindestens einer der in den mindestens zwei parallel geschalteten Reaktoren enthaltenen Katalysatoren während des Verfahrens regeneriert wird.

3. Verfahren nach Anspruch 2, **dadurch gekennzeichnet, daß** die Katalysator-Regenerierung derart durchgeführt wird, daß mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Reaktionsstufe immer mindestens ein Reaktor zur Umsetzung von organischer Verbindung mit Hydroperoxid zur Verfügung steht.

4. Verfahren nach Anspruch 2 oder 3, **dadurch gekennzeichnet, daß** von x parallel geschalteten Reaktoren einer Reaktionsstufe immer x-1 Reaktoren zur Umsetzung von organischer Verbindung mit Hydroperoxid zur Verfügung stehen.

5. Verfahren nach einem der Ansprüche 2 bis 4, umfassend mindestens eine weitere Zwischenbehandlung und mindestens eine weitere Reaktionsstufe.

6. Verfahren nach einem der Ansprüche 2 bis 5, **dadurch gekennzeichnet, daß** mindestens eine Zwischenbehandlung
- eine destillative Abtrennung des Hydroperoxides aus mindestens einem Produktstrom oder
- eine Zugabe einer Base zu mindestens einem Produktstrom
ist.

7. Verfahren nach einem der Ansprüche 2 bis 6, **dadurch gekennzeichnet, daß**
- in der Reaktionsstufe (R1) Hydroperoxid mit organischer Verbindung in mindestens zwei parallel geschalteten Reaktoren in Anwesenheit eines Katalysators unter Erhalt mindestens eines Produktstromes (P1), umfassend nicht umgesetztes Hydroperoxid, nicht umgesetzte organische Verbindung und umgesetzte organische Verbindung, umgesetzt wird,
- in der Zwischenbehandlung (Z1) zu dem mindestens einen Produktstrom (P1) Base unter Erhalt mindestens eines Produktstromes (PZ1) zugegeben wird,
- in der Reaktionsstufe (R2) nicht umgesetztes Hydroperoxid und nicht umgesetzte organische Verbindung, enthalten in dem mindestens einen Produktstrom (PZ1), unter Erhalt mindestens eines Produktstromes (P2), umfassend nicht umgesetztes Hydroperoxid, in mindestens zwei parallel geschalteten Reaktoren umgesetzt werden,
- in einer Zwischenbehandlung (Z2) aus dem mindestens einen Produktstrom (P2) nicht umgesetztes Hydroperoxid unter Erhalt mindestens eines Produktstromes (PZ2), umfassend Hydroperoxid, destillativ abgetrennt wird, und
- das Hydroperoxid, enthalten in dem mindestens einen Produktstrom (PZ2), in einer Reaktionsstufe (R3) mit organischer Verbindung unter Erhalt mindestens eines Produktstroms (P3) umgesetzt wird.

8. Verfahren nach einem der Ansprüche 1 bis 7, **dadurch gekennzeichnet, daß** als Reaktoren Festbettreaktoren eingesetzt werden.

9. Verfahren nach einem der Ansprüche 1 bis 8, **dadurch gekennzeichnet, daß** Wasserstoffperoxid mit Alken zu Alkenoxid umgesetzt wird, wobei als Katalysator ein Zeolith-Katalysator eingesetzt wird.

10. Verfahren nach Anspruch 9, **dadurch gekennzeichnet, dass** Propen zu Propylenoxid an einem Titan-Silikalit-Katalysator umgesetzt wird.

11. Verfahren nach Anspruch 10, **dadurch gekennzeichnet, dass** als Katalysator ein Titan-Silikalit-Katalysator der Struktur TS-1 eingesetzt wird.

12. Vorrichtung zur Durchführung eines Verfahrens wie in einem der Ansprüche 1 bis 11 definiert, umfassend mindestens zwei parallel geschaltete Reaktoren für die Reaktionsstufe (R1), mindestens eine für die Zwischenbehandlung (Z1) vorgesehenene Vorrichtung ausgewählt aus der Gruppe umfassend Reaktor, Abtrennvorrichtung oder Mischvorrichtung, und mindestens zwei parallel geschaltete Reaktoren für die Reaktionsstufe (R2).

## Claims

1. A continuous process for reacting an organic compound with hydroperoxide in the presence of a catalyst, wherein
- the organic compound is reacted with hydroperoxide in the presence of a catalyst in a reaction stage (R1) to give at least one product stream (P1),
- the product stream or streams (P1) is passed to an intermediate treatment (I1) to give, as output from (I1), at least one product stream (PI1) comprising hydroperoxide, and
- the product stream or streams (PI1) is passed to a reaction stage (R2) in which hydroperoxide is reacted with the organic compound in the presence of a catalyst to give at least one product stream (P2),
wherein a reactor assembly comprising at least two reactors connected in parallel is used in each of the reaction stages (R1) and (R2).

2. A process as claimed in claim 1, wherein at least one of the catalysts present in the two or more reactors connected in parallel is regenerated during the process.

3. A process as claimed in claim 2, wherein the catalyst regeneration is carried out in such a way that at least one of the parallel reactors is decoupled from the respective reaction stage and the catalyst present in this reactor is regenerated, so that at least one reactor in each reaction stage is always available for the reaction of organic compound with hydroperoxide during the course of the continuous process.

4. A process as claimed in claim 2 or 3, wherein x-1 reactors of x reactors connected in parallel in a reaction stage are always available for the reaction of organic compound with hydroperoxide.

5. A process as claimed in any of claims 2 to 4 comprising at least one further intermediate treatment and at least one further reaction stage.

6. A process as claimed in any of claims 2 to 5, wherein at least one intermediate treatment is
- separation of the hydroperoxide from at least one product stream by distillation or
- addition of a base to at least one product stream.

7. A process as claimed in any of claims 2 to 6, wherein
- in the reaction stage (R1), hydroperoxide is reacted with organic compound in at least two reactors connected in parallel in the presence of a catalyst to give at least one product stream (P1) comprising unreacted hydroperoxide, unreacted organic compound and reacted organic compound,
- in the intermediate treatment (I1), base is added to the product stream or streams (P1) to give at least one product stream (PI1),
- in the reaction stage (R2), unreacted hydroperoxide and unreacted organic compound present in the product stream or streams (PI1) are reacted in at least two reactors connected in parallel to give at least one product stream (P2) comprising unreacted hydroperoxide,
- in an intermediate treatment (I2), unreacted hydroperoxide is separated by distillation from the product stream or streams (P2) to give at least one product stream (PI2) comprising hydroperoxide, and
- the hydroperoxide present in the product stream or streams (PI2) is reacted with organic compound in a reaction stage (R3) to give at least one product stream (P3).

8. A process as claimed in any of claims 1 to 7, wherein fixed-bed reactors are used as reactors.

9. A process as claimed in any of claims 1 to 8, wherein hydrogen peroxide is reacted with alkene to give alkene oxide, using a zeolite catalyst as catalyst.

10. A process as claimed in claim 9, wherein propene is converted into propylene oxide over a titanium silicalite catalyst.

11. A process as claimed in claim 10, wherein the catalyst used is a titanium silicalite catalyst having the TS-1 structure.

12. An apparatus for carrying out a process as defined in any of claims 1 to 11, which comprises at least two reactors connected in parallel for the reaction stage (R1), at least one apparatus selected from the group consisting of reactor, separation apparatus and mixing apparatus provided for the intermediate treatment (I1) and at least two reactors connected in parallel for the reaction stage (R2).

## Revendications

1. Procédé continu pour faire réagir un composé organique avec de l'hydroperoxyde, en présence d'un catalyseur, dans lequel
- le composé organique est, dans une étape réactionnelle (R1), mis à réagir avec de l'hydroperoxyde en présence d'un catalyseur avec obtention d'au moins un courant de produit (P1),
- ledit au moins un courant de produit (P1) est amené à un traitement intermédiaire (Z1), au moins un courant de produit (PZ1), comportant de l'hydroperoxyde, étant obtenu à partir de (Z1), et
- ledit au moins un courant de produit (PZ1) est amené à une étape réactionnelle (R2) dans laquelle de l'hydroperoxyde est mis à réagir avec le composé organique en présence d'un catalyseur, avec obtention d'au moins un courant de produit (P2),
**caractérisé en ce que**, dans les étapes réactionnelles (R1) et (R2), un agencement de réacteurs comportant au moins deux réacteurs montés en parallèle est chaque fois mis en oeuvre.

2. Procédé suivant la revendication 1, **caractérisé en ce qu'**au moins un des catalyseurs contenus dans les au moins deux réacteurs montés en parallèle est régénéré pendant le procédé.

3. Procédé suivant la revendication 2, **caractérisé en ce que** la régénération du catalyseur est effectuée de telle façon qu'au moins un des réacteurs montés en parallèle soit découplé de l'étape réactionnelle respective et que le catalyseur contenu dans ce réacteur soit régénéré, au moins un réacteur étant toujours disponible, dans chaque étape réactionnelle, pour la réaction de composé organique avec de l'hydroperoxyde au cours du procédé continu.

4. Procédé suivant l'une des revendications 2 et 3, **caractérisé en ce que**, parmi x réacteurs montés en parallèle d'une étape réactionnelle, sont toujours disponibles x-1 réacteurs pour la réaction de composé organique avec de l'hydroperoxyde.

5. Procédé suivant l'une des revendications 2 à 4, comprenant au moins un traitement intermédiaire supplémentaire et au moins une étape réactionnelle supplémentaire.

6. Procédé suivant l'une des revendications 2 à 5, **caractérisé en ce qu'**au moins un traitement intermédiaire est
- une séparation par distillation de l'hydroperoxyde à partir d'au moins un courant de produit, ou
- une addition d'une base à au moins un courant de produit.

7. Procédé suivant l'une des revendications 2 à 6, **caractérisé en ce que**
- dans l'étape réactionnelle (R1), de l'hydroperoxyde est mis à réagir avec un composé organique dans au moins deux réacteurs montés en parallèle, en présence d'un catalyseur, avec obtention d'au moins un courant de produit (P1) comportant de l'hydroperoxyde qui n'a pas réagi, du composé organique qui n'a pas réagi et du composé organique qui a réagi,
- dans le traitement intermédiaire (Z1), une base est ajoutée à au moins un courant de produit (P1), avec obtention d'au moins un courant de produit (PZ1),
- dans l'étape réactionnelle (R2), de l'hydroperoxyde qui n'a pas réagi et du composé organique qui n'a pas réagi, contenus dans le au moins un courant de produit (PZ1), sont mis à réagir dans au moins deux réacteurs montés en parallèle, avec obtention d'au moins un courant de produit (P2) comportant de l'hydroperoxyde qui n'a pas réagi,
- dans un traitement intermédiaire (Z2), on sépare par distillation à partir du au moins un courant de produit (P2) de l'hydroperoxyde qui n'a pas réagi, avec obtention d'au moins un courant de produit (PZ2), comportant de l'hydroperoxyde, et
- l'hydroperoxyde, contenu dans le au moins un courant de produit (PZ2), est mis à réagir dans une étape réactionnelle (R3) avec un composé organique, avec obtention d'au moins un courant de produit (P3).

8. Procédé suivant l'une des revendications 1 à 7, **caractérisé en ce que**, comme réacteurs, on met en oeuvre des réacteurs à lit fixe.

9. Procédé suivant l'une des revendications 1 à 8, **caractérisé en ce qu'**on met à réagir du peroxyde d'hydrogène avec un alcène pour former de l'oxyde d'alcène, un catalyseur à base de zéolite étant mis en oeuvre comme catalyseur.

10. Procédé suivant la revendication 9, **caractérisé en ce qu'**on fait réagir du propène en oxyde de propylène sur un catalyseur à base de titane-silicalite.

11. Procédé suivant la revendication 10, **caractérisé en ce que**, comme catalyseur, on met en oeuvre un catalyseur à base de titane-silicalite de la structure TS-1.

12. Dispositif pour la mise en oeuvre d'un procédé tel que défini dans l'une des revendications 1 à 11, comportant au moins deux réacteurs montés en parallèle pour l'étape réactionnelle (R1), au moins un dispositif prévu pour le traitement intermédiaire (Z1) choisi parmi le groupe comprenant un réacteur, un dispositif de séparation ou un dispositif de mélange, et au moins deux réacteurs montés en parallèle pour l'étape réactionnelle (R2).
